# EUROPÄISCHE PATENTSCHRIFT

(11) **EP 3 269 410 B1**
(45) Veröffentlichungstag und Bekanntmachung des Hinweises auf die Patenterteilung: **01.04.2020**
(21) Anmeldenummer: 17176793.2
(22) Anmeldetag: 20.06.2017
(51) Int. Cl.: A61M 5/142, A61M 5/148, A61M 5/152

(54) **MOBILE INFUSIONSPUMPE**
MOBILE INFUSION PUMP
POMPE D'INFUSION MOBILE

(30) Priorität: 11.07.2016 DE 102016212579
(43) Veröffentlichungstag der Anmeldung: 17.01.2018
(73) Patentinhaber: Raumedic AG, 95213 Münchberg (DE)
(72) Erfinder: Prescher, Jörg, 97729 Ramsthal (DE); Moreth, Daniel, 95028 Hof (DE); Gläsel, Björn, 95158 Kirchenlamitz (DE)
(74) Vertreter: Rau, Schneck & Hübner Patentanwälte Rechtsanwälte PartGmbB

(56) Entgegenhaltungen:
- DE-A1- 3 021 533
- US-A- 3 062 153
- US-A1- 2004 168 723
- US-A1- 2007 156 103
- US-A1- 2012 016 306
- US-B1- 8 177 740

## Beschreibung

Die Erfindung betrifft eine mobile Infusionspumpe zur gleichmäßigen Abgabe eines fließfähigen Mediums. Ferner betrifft die Erfindung einen Ballonkörper für eine derartige Infusionspumpe sowie einen Schlauchkörper als Halbzeug zum Einsatz als Bestandteil eines derartigen Ballonkörpers.

Mobile Infusionspumpen kommen in der ambulanten medizinischen Behandlung, insbesondere bei der künstlichen Ernährung, Chemotherapie und Schmerztherapie, zum Einsatz. Herkömmliche Infusionspumpen verfügen über einen Ballonkörper zur Aufnahme des fließfähigen Mediums. Der Ballonkörper wird über eine Zugabeeinheit mit dem fließfähigen Medium gefüllt und dehnt sich hierbei aus. Durch die Ausdehnung des Ballonkörpers werden in diesem Rückstellkräfte erzeugt, welche die Abgabe des fließfähigen Mediums unter Druck ermöglichen. Die Abgabe wird hierbei über eine Abgabeeinheit, welche eine Fluidverbindung zwischen der Infusionspumpe und einem Patienten darstellt, durchgeführt. Derartige Infusionspumpen werden beispielsweise in den Druckschriften US 8,398,595 B2, US 7,341,572, US 2004/0168723 A1, US 2012/0016306 A1, US 8,177,740 B1, US 2007/0156103 A1 und US 8,439,862 offenbart. Die US 3,062,153 beschreibt eine Pumpe für insbesondere industrielle Anwendungen. Die DE 30 21 533 A1 betrifft einen Schlauch für einen Bowdenzug.

Die Aufgabe der vorliegenden Erfindung ist es daher, eine Infusionspumpe bereitzustellen, die ein präzises Dosierverhalten bei der Abgabe des fließfähigen Mediums aufweist. Insbesondere soll eine Variation der Gesamtdauer des Abgabeprozesses höchstens 10 % einer Soll-Abgabedauer betragen.

Diese Aufgabe ist erfindungsgemäß gelöst durch eine mobile Infusionspumpe mit den im Anspruch 1 angegebenen Merkmalen.

Die erfindungsgemäße mobile Infusionspumpe umfasst einen Ballonkörper zur Aufnahme des Mediums, eine Zugabeeinheit sowie eine Abgabeeinheit. Zugabe- und Abgabeeinheit stehen in Fluidverbindung mit einem inneren Volumen des Ballonkörpers zur Zugabe bzw. Abgabe des fließfähigen Mediums in bzw. aus dem Ballonkörper. Zur Vorgabe einer Abgabe-Fließrate des fließfähigen Mediums ist in der Abgabeeinheit weiterhin eine Drosseleinheit vorgesehen. Um das Ausdehnverhalten des Ballonkörpers und damit das Dosierverhalten der Infusionspumpe zu optimieren, ist es vorgesehen, dass ein Teil des Ballonkörpers als Schlaukörper mit einem Querschnittsprofil mit mehreren durchgehenden Längsrippen ausgeführt ist. Die Längsrippen erhöhen die Stabilität des Schlauchkörpers. Hierdurch wird das Ausdehnverhalten gezielt gesteuert, da sich nur einzelne, zwischen den Längsrippen befindliche Segmente ausdehnen können. Das gesteuerte Ausdehnverhalten des Ballonkörpers gewährleistet einen gleichmäßigen Abgabedruck, wodurch das Dosierverhalten und die Dauer des Abgabeprozesses genauer definiert werden. Eine unerwünscht ungleichmäßige Ausdehnung des Ballonkörpers, die in einem ungleichmäßigen Abgabedruck resultieren würde, ist vermieden. Entsprechend ergibt sich keine unerwünschte Variation der Abgabedauer.

Die erhöhte Stabilität des Schlauchkörpers hat weiterhin den Vorteil einer vereinfachten Handhabung und Montage des Ballonkörpers in der Infusionspumpe.

Die Ausführung mindestens einer der Längsrippen als Innen-Längsrippe gemäß Anspruch 2 ermöglicht es, das Aufliegen des Ballonkörpers auf einem Stützkörper der Infusionspumpe gezielt zu steuern. Hierdurch wird ein Anhaften des Ballonkörpers an dem Stützkörper, wie es zum Beispiel bei längerer Lagerung der Infusionspumpe vorkommen kann, verhindert.

Durch das Vorsehen mindestens zweier Innen-Längsrippen können die Stützkräfte der Innen-Längsrippen entlang einer Innenprofilierung des Schlauchkörpers besser verteilt werden. Auch wird die Auflagefläche des Schlauchkörpers auf dem Stützkörper weiter verkleinert. In den Zwischenräumen zwischen den Längsrippen kann bei Abgabe ein Teil des fließfähigen Mediums verbleiben. Eine Restentleerung des Ballonkörpers kann dadurch verhindert werden.

Die Ausgestaltung nach Anspruch 4, wonach eine Außenprofilierung des Schlauchkörpers durch mindestens eine als Außen-Längsrippe ausgeführte Längsrippe erzeugt wird, hat den Vorteil, dass die Stabilität des Schlauchkörpers erhöht wird, ohne eine Restentleerung des Ballonkörpers bei der Abgabe des fließfähigen Mediums zu verhindern.

Eine Mehrzahl von Außen-Längsrippen gemäß dem Anspruch 5 verbessert die Verteilung der Stützkräfte entlang eines Außenumfanges des Schlauchkörpers.

Eine Infusionspumpe mit den Merkmalen des Anspruchs 6 kombiniert Innen-Längsrippen und Außen-Längsrippen. Durch die in Umfangsrichtung auf gleicher Höhe angeordneten Innen- und Außen-Längsrippen wird die Stabilität der jeweiligen Längsrippen verbessert. Die Stützkräfte der Längsrippen sind daher lokal erhöht.

Auch eine Infusionspumpe nach Anspruch 7 kombiniert Innen- und Außen-Längsrippen. Eine in Umfangsrichtung des Schlauchkörpers zueinander versetzte Anordnung der Innen- und Außen-Längsrippen gewährleistet eine optimale Verteilung der Stützkräfte.

Des Weiteren betrifft die Erfindung einen Ballonkörper für eine Infusionspumpe, wie sie vorstehend umrissen wurde. Ein weiterer Gegenstand der Erfindung ist ein Schlauchkörper als Halbzeug zum Einsetzen als Bestandteil eines solchen Ballonkörpers.

Ein Ausführungsbeispiel der Erfindung wird nachfolgend anhand der Zeichnungen näher erläutert. In diesen zeigen:
- Fig. 1: eine schematische Darstellung einer mobilen Infusionspumpe zur gleichmäßigen Abgabe eines fließfähigen Mediums, und
- Fig. 2 bis 5: Querschnitte durch alternative Ausführungsformen eines Schlauchkörpers als Teil eines Ballonkörpers zur Verwendung in der Infusionspumpe.

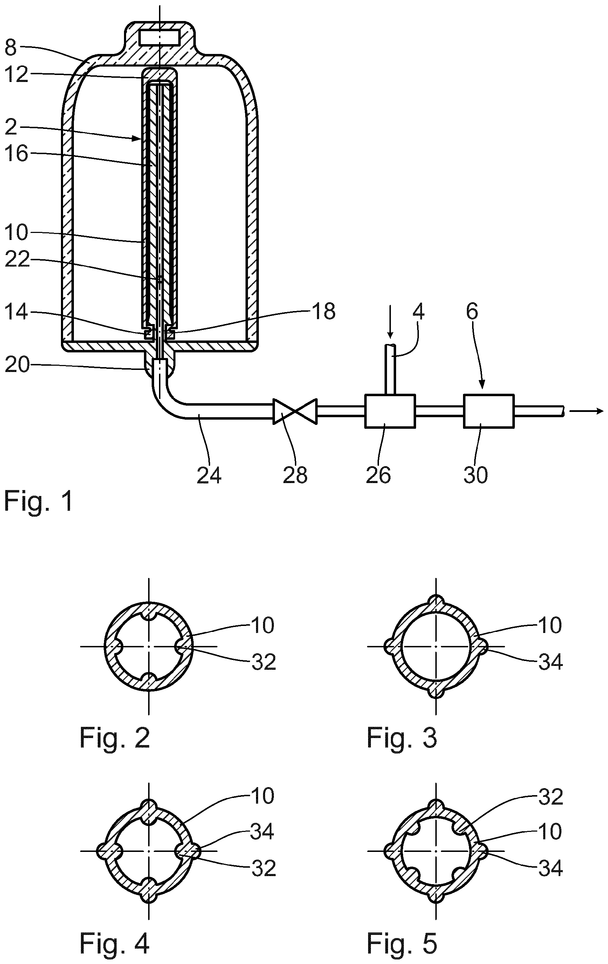

Fig. 1 zeigt eine mobile Infusionspumpe zur gleichmäßigen Abgabe eines fließfähigen Mediums. Die Infusionspumpe umfasst eine Ballonkörper 2 zur Aufnahme des fließfähigen Mediums, eine Zugabeeinheit 4 und eine Abgabeeinheit 6. Zur Zugabe bzw. Abgabe des fließfähigen Mediums stehen die Zugabeeinheit 4 und die Abgabeeinheit 6 in einer weiteren, unten beschriebenen Fluidverbindung mit einem inneren Volumen des Ballonkörpers 2.

Um den Ballonkörper 2 vor äußeren Einflüssen zu schützen, ist dieser von einem festen Außengehäuse 8 umschlossen. Um den Füllzustand des Ballonkörpers 2 jederzeit überprüfen zu können, ist das Außengehäuse 8 transparent.

Der Ballonkörper 2 umfasst einen Schlauchkörper 10, einen geschlossenen Endabschnitt 12 sowie einen den Endabschnitt 12 gegenüberliegenden Kragen 14. Der Ballonkörper 2 umschließt einen zylindrischen Stützkörper 16, dessen Längsachse 17 mit der Längsachse des Schlauchkörpers 10 übereinstimmt. Der Kragen 14 des Ballonkörpers 2 umgreift einen verjüngten Halsabschnitt 18 des Stützkörpers 16. Der Stützkörper 16 liegt also vollständig im Innenvolumen des Ballonkörpers 2. Durch den Kragen 14 wird das Innenvolumen des Ballonkörpers 2 gegenüber dem restlichen Gehäuse abgedichtet und der Ballonkörper 2 auf dem Stützkörper 16 gehalten. Der Stützkörper 16 ist mit dem Außengehäuse 8 verbunden. In Verlängerung des Stützkörpers 16 ist ein Schlauchanschluss 20 am Außengehäuse angeordnet. Der Stützkörper 16 ist als ein mit dem Schlauchanschluss 20 in Fluidverbindung stehender Hohlkörper ausgeführt. Eine Fluidverbindung zwischen dem Hohlkörper des Stützkörpers 16 und dem Innenvolumen des Ballonkörpers 2 wird durch Radialbohrungen 22 durch den Zylindermantel des Stützkörpers 16 gewährleistet.

In dem dargestellten Ausführungsbeispiel weist der Schlauchanschluss einen Schlauch 24 als Teil der Fluidverbindung zwischen dem Innenvolumen des Ballonkörpers 2 und der Zugabeeinheit 4 bzw. der Abgabeeinheit 6 auf. An dem dem Schlauchanschluss 20 gegenüberliegenden Ende des Schlauches 24 ist eine Kupplung 26 angeordnet. Mittels der Kupplung 26 kann der Schlauch 24 wahlweise mit der Zugabeeinheit 4 zur Zugabe des fließfähigen Mediums oder mit der Abgabeeinheit 6 zur Abgabe des fließfähigen Mediums in Fluidverbindung gebracht werden. Am Schlauch 24 ist ein Ventil 28 zum Verschließen der Fluidverbindung vorgesehen. Das Ventil 28 kann insbesondere als Schlauchklemme ausgeführt sein. Die Fluidverbindung zwischen dem Innenvolumen des Ballonkörpers 2 und der Zugabeeinheit 4 bzw. der Abgabeeinheit 6 umfasst also die Kupplung 26, den Schlauch 24 mit Ventil 28, den Schlauchanschluss 20 sowie den als Hohlkörper ausgeführten Stützkörper 16. Alternativ zu der hier gezeigten Ausführung mit einem einzigen Schlauch 24 und einer Kupplung 26 könnten auch getrennte Fluidverbindungen für die Zugabeeinheit 4 und die Abgabeeinheit 6 vorgesehen sein.

Die Zugabeeinheit 4 ist in Fig. 1 nicht näher dargestellt. Als Zugabeeinheit 4 kann beispielsweise eine über einen Spritzenanschluss verbundene Spritze vorgesehen sein, durch welche das fließfähige Medium in den Ballonkörper 2 injiziert wird. Alternativ hierzu könnten auch andersartige Zugabeeinheiten vorgesehen sein.

Die stromabwärts der Kupplung 26 angeordnete Abgabeeinheit 6 weist eine Drosseleinheit 30 zur Vorgabe einer Abgabe-Fließrate des fließfähigen Mediums auf. Die weitere Abgabeeinheit 6 ist nicht explizit bei der Fig. 1 gezeigt. Sie umfasst eine Fluidverbindung zum Patienten, beispielsweise über einen intramuskulären Zugang.

Der Ballonkörper 2 ist aus einem elastischen Material geformt. In entleertem Zustand ist das Innenvolumen des Ballonkörpers 2 minimal. Es ist jedoch mindestens so groß, wie das Volumen des Stützkörpers 16. Bei der Zugabe des fließfähigen Mediums strömt dieses durch die Radialbohrung 22 des Stützkörpers 16 in das Innenvolumen des Ballonkörpers 2 und dehnt diesen aus. Nach Beendigung der Zugabe ist das Innenvolumen des Ballonkörpers 2 um das Volumen des zugegebenen fließfähigen Mediums ausgedehnt. Das Volumen des zugegebenen fließfähigen Mediums kann hierbei an die Behandlung und den Bedarf des Patienten angepasst werden. Es kann beispielsweise 75 ml oder 100 ml betragen.

Durch die Ausdehnung des elastischen Materials des Ballonkörpers 2 wird eine Rückstellkraft erzeugt, welche bei der Abgabe des fließfähigen Mediums als Pumpendruck dient. Um einen über den Abgabezeitraum möglichst konstanten Pumpendruck zu erzeugen, ist es notwendig, dass der Ballonkörper 2 bei der Zugabe des fließfähigen Mediums gleichmäßig ausgedehnt wird. Um eine möglichst gleichmäßige Ausdehnung des Ballonkörpers 2 zu gewährleisten, sind Längsrippen am Schlauchkörper 10 des Ballonkörpers 2 vorgesehen. Die Längsrippen verlaufen entlang der Längsachse des Schlauchkörpers 10 durchgehend über dessen gesamte Länge. Die Längsrippen stabilisieren den Schlauchkörper 10 entlang einer Längsachse. Hierdurch können nur einzelne Segmente zwischen den Längsrippen ausgedehnt werden, wodurch das Ausdehnverhalten des Ballonkörpers 2 und folglich das Dosierverhalten der Infusionspumpe verbessert wird.

In Fig. 2 bis 5 sind alternative Ausführungsformen von Schlauchkörpern 10 mit verschiedenen Anordnungen von Längsrippen dargestellt. Hierzu zeigen die Fig. 2 bis 5 Querschnitte senkrecht zu der Längsachse der verschiedenen Schlauchkörper 10.

In Fig. 2 ist ein Schlauchkörper 10 gezeigt, bei welchem vier Längsrippen als Innen-Längsrippen 32 einen Teil einer Innenprofilierung des Schlauchkörpers 10 darstellen. Zusätzlich zu einer erhöhten Stabilität des Schlauchkörpers 10 hat dies den Vorteil, dass eine Auflagefläche der Innenprofilierung des Schlauchkörpers 10 auf den Stützkörper 16 minimiert wird. Hierdurch kann ein Anhaften des Schlauchkörpers 10 am Stützkörper 16 vermieden werden. Ein derartiges Anhaften kann das Ausdehnverhalten des Ballonkörpers 2 nachteilig beeinflussen.

Bei der in Fig. 2 dargestellten Variante mit Innen-Längsrippen 32 verbleibt bei Abgabe des fließfähigen Mediums ein Rest des fließfähigen Mediums in den Zwischenräumen zwischen den Innen-Längsrippen 32 und wird nicht an den Patienten abgegeben. Möchte man dies vermeiden, können, wie in Fig. 3 gezeigt, auch Längsrippen vorgesehen sein, welche als Außen-Längsrippen 34 Teil einer Außenprofilierung des Schlauchkörpers 10 sind.

Eine weitere Verbesserung der Stabilität und des Ausdehnverhaltens des Ballonkörpers 2 kann durch eine Kombination von Innen-Längsrippen 32 und Außen-Längsrippen 34 erreicht werden. Wie in Fig. 4 dargestellt, können die Innen-Längsrippen 32 hierbei in Umfangsrichtung des Schlauchkörpers 10 auf gleicher Höhe angeordnet sein wie die Außen-Längsrippen 34. Durch die sich jeweils gegenüberliegenden Außen-Längsrippen 34 und Innen-Längsrippen 32 verstärken sich diese jeweils, wodurch die Stabilität des Schlauchkörpers 10 entlang der Längsrippen erhöht wird. Alternativ hierzu können die Innen-Längsrippen 32 und die Außen-Längsrippen 34 in Umfangsrichtung des Schlauchkörpers 10 zueinander versetzt angeordnet sein (Fig. 5). Hierdurch werden die Stützkräfte der Längsrippen noch besser verteilt und ein Ausdehnverhalten des Ballonkörpers weiter optimiert.

## Patentansprüche

1. Mobile Infusionspumpe zur gleichmäßigen Abgabe eines fließfähigen Mediums umfassend
a. einen dehnbaren Ballonkörper (2) zur Aufnahme des Mediums,
b eine Zugabeeinheit (4), die in Fluidverbindung mit einem inneren Volumen des Ballonkörpers (2) steht, zur Zugabe des Mediums in den Ballonkörper (2), und
c. eine Abgabeeinheit (6), die in Fluidverbindung mit einem inneren Volumen des Ballonkörpers (2) steht, zur Abgabe des Mediums aus dem Ballonkörper (2),
d. wobei die Abgabeeinheit (6) eine Drosseleinheit (30) zur Vorgabe einer Abgabe-Fließrate des Mediums aufweist,
**dadurch gekennzeichnet, dass**
e. ein Teil des dehnbaren Ballonkörpers (2) ein Schlauchkörper (10) mit einem Querschnittsprofil mit mehreren durchgehenden Längsrippen (32; 34) ist.

2. Infusionspumpe nach Anspruch 1, **dadurch gekennzeichnet, dass** mindestens eine der Längsrippen (32; 34) als Innen-Längsrippe (32) Teil einer Innenprofilierung des Schlauchkörpers (10) ist.

3. Infusionspumpe nach Anspruch 2, **dadurch gekennzeichnet, dass** mindestens zwei Innen-Längsrippen (32) vorgesehen sind.

4. Infusionspumpe nach einem der vorgenannten Ansprüche, **dadurch gekennzeichnet, dass** mindestens eine der Längsrippen (32; 34) als Außen-Längsrippe (34) Teil einer Außenprofilierung des Schlauchkörpers (10) ist.

5. Infusionspumpe nach Anspruch 4, **dadurch gekennzeichnet, dass** mindestens zwei, insbesondere mindestens vier, Außen-Längsrippen (34) vorgesehen sind.

6. Infusionspumpe nach Anspruch 1, **dadurch gekennzeichnet, dass** mindestens eine Innen-Längsrippe (32) als Teil einer Innenprofilierung des Schlauchkörpers (10) und mindestens eine Außen-Längsrippe (34) als Teil einer Außenprofilierung des Schlauchkörpers (10) vorgesehen sind, wobei die mindestens eine Innen-Längsrippe (32) in Umfangsrichtung des Schlauchkörpers (10) auf gleicher Höhe angeordnet ist wie die mindestens eine Außen-Längsrippe (34).

7. Infusionspumpe nach Anspruch 1, **dadurch gekennzeichnet, dass** mindestens eine Innen-Längsrippe (32) als Teil einer Innenprofilierung des Schlauchkörpers (10) und mindestens eine Außen-Längsrippe (34) als Teil einer Außenprofilierung des Schlauchkörpers (10) vorgesehen sind, die in Umfangsrichtung des Schlauchkörpers (10) zueinander versetzt angeordnet sind.

8. Dehnbarer Ballonkörper (2) für eine Infusionspumpe nach einem der vorgenannten Ansprüche, **dadurch gekennzeichnet, dass** ein Teil des dehnbaren Ballonkörpers (2) ein Schlauchkörper (10) mit einem Querschnittsprofil mit mehreren durchgehenden Längsrippen (32; 34) ist.

9. Schlauchkörper (10) als Halbzeug zum Einsatz als Bestandteil eines dehnbaren Ballonkörpers (2) nach Anspruch 8, **gekennzeichnet durch** ein Querschnittsprofil mit mehreren durchgehenden Längsrippen (32; 34).

## Claims

1. Mobile infusion pump for the constant delivery of a fluid medium, the mobile infusion pump comprising
a. an expandable balloon body (2) for receiving the medium,
b. a filling unit (4), which is in fluid connection with an internal volume of the balloon body (2), for filling the medium into the balloon body (2), and
c. a delivery unit (6), which is in fluid connection with an internal volume of the balloon body (2), for delivering the medium from the balloon body (2),
d. wherein the delivery unit (6) is provided with a throttling unit (30) for defining a delivery flow rate of the medium,
**characterized in that**
e. a part of the expandable balloon body (2) is a tube body (10) with a cross-sectional profile comprising a plurality of continuous longitudinal ribs (32; 34).

2. Infusion pump according to claim 1, **characterized in that** at least one of the longitudinal ribs (32; 34) is configured as an internal longitudinal rib (32) forming part of an internal profile of the tube body (10).

3. Infusion pump according to claim 2, **characterized in that** at least two internal longitudinal ribs (32) are provided.

4. Infusion pump according to any one of the preceding claims, **characterized in that** at least one of the longitudinal ribs (32; 34) is configured as an external longitudinal rib (34) forming part of an external profile of the tube body (10).

5. Infusion pump according to claim 4, **characterized in that** at least two, in particular at least four, external longitudinal ribs (34) are provided.

6. Infusion pump according to claim 1, **characterized in that** at least one internal longitudinal rib (32) is provided as part of an internal profile of the tube body (10) and at least one external longitudinal rib (34) is provided as part of an external profile of the tube body (10), wherein the at least one internal longitudinal rib (32) is arranged on a level with the at least one external longitudinal rib (34) when seen in a circumferential direction of the tube body (10).

7. Infusion pump according to claim 1, **characterized in that** at least one internal longitudinal rib (32) is provided as part of an internal profile of the tube body (10) and at least one external longitudinal rib (34) is provided as part of an external profile of the tube body (10), the at least one internal longitudinal rib (32) and the at least one external longitudinal rib (34) being arranged such as to be staggered relative to each other when seen in the circumferential direction of the tube body (10).

8. Expandable balloon body (2) for an infusion pump according to any one of the preceding claims, **characterized in that** a part of the expandable balloon body (2) is a tube body (10) with a cross-sectional profile comprising a plurality of continuous longitudinal ribs (32; 34).

9. Tube body (10) as a semi-finished product to be used as a component of an expandable balloon body (2) according to claim 8, **characterized by** a cross-sectional profile having a plurality of continuous longitudinal ribs (32; 34).

## Revendications

1. Pompe à perfusion mobile pour la distribution régulière d'un milieu coulant comprenant
a. un corps de ballonnet (2) extensible pour le logement du milieu,
b. une unité d'ajout (4), qui est en liaison fluidique avec un volume intérieur du corps de ballonnet (2), pour l'ajout du milieu dans le corps de ballonnet (2), et
c. une unité de distribution (6), qui est en liaison fluidique avec un volume intérieur du corps de ballonnet (2), pour l'ajout du milieu à partir du corps de ballonnet (2),
d. dans laquelle l'unité de distribution (6) présente une unité d'étranglement (30) destinée à prédéfinir un débit de distribution du milieu,
**caractérisée en ce que**
e. une partie du corps de ballonnet (2) extensible est un corps de tuyau (10) avec un profil de section transversale avec plusieurs nervures longitudinales (32 ; 34) continues.

2. Pompe à perfusion selon la revendication 1, **caractérisée en ce qu'**au moins une des nervures longitudinales (32 ; 34) en tant que nervure longitudinale intérieure (32) fait partie d'un profilage intérieur du corps de tuyau (10).

3. Pompe à perfusion selon la revendication 2, **caractérisée en ce qu'**au moins deux nervures longitudinales intérieures (32) sont prévues.

4. Pompe à perfusion selon l'une quelconque des revendications précédentes, **caractérisée en ce qu'**au moins une des nervures longitudinales (32 ; 34) en tant que nervure longitudinale extérieure (34) fait partie d'un profilage extérieur du corps de tuyau (10).

5. Pompe à perfusion selon la revendication 4, **caractérisée en ce qu'**au moins deux, en particulier au moins quatre nervures longitudinales extérieures (34) sont prévues.

6. Pompe à perfusion selon la revendication 1, **caractérisée en ce qu'**au moins une nervure longitudinale intérieure (32) fait partie d'un profilage intérieur du corps de tuyau (10) et au moins une nervure longitudinale extérieure (34) en tant que partie d'un profilage extérieur du corps de tuyau (10) sont prévues, dans laquelle la au moins une nervure longitudinale intérieure (32) est disposée dans la direction périphérique du corps de tuyau (10) à la même hauteur que la au moins une nervure longitudinale extérieure (34).

7. Pompe à perfusion selon la revendication 1, **caractérisée en ce qu'**au moins une nervure longitudinale intérieure (32) en tant que partie d'un profilage intérieur du corps de tuyau (10) et au moins une nervure longitudinale extérieure (34) en tant que partie d'un profilage extérieur du corps de tuyau (10) sont prévues, qui sont disposées dans la direction périphérique du corps de tuyau (10) de manière décalée l'une de l'autre.

8. Corps de ballonnet (2) extensible pour une pompe à infusion selon l'une quelconque des revendications précédentes, **caractérisé en ce qu'**une partie du corps de ballonnet (2) extensible est un corps de tuyau (10) avec un profil de section transversale avec plusieurs nervures longitudinales (32 ; 34) continues.

9. Corps de ballonnet (2) en tant que demi-produit destiné à être utilisé en tant que constituant d'un corps de ballonnet (2) extensible selon la revendication 8, **caractérisé par** un profil en section transversale avec plusieurs nervures longitudinales (32 ; 34) continues.
